# EUROPÄISCHE PATENTANMELDUNG

(11) **EP 0 924 494 A2**
(43) Veröffentlichungstag der Anmeldung: **23.06.1999**
(21) Anmeldenummer: 98124070.8
(22) Anmeldetag: 18.12.1998
(51) Int. Cl.: G01B 11/24

(54) **Topometer für spiegelnde Flächen**

(30) Priorität: 20.12.1997 DE 19757106
(71) Anmelder: Massig, Jürgen Prof. Dr., 73457 Essingen (DE); Lingelbach, Bernd Prof. Dr., 73453 Abtsgmünd (DE)
(72) Erfinder: Massig, Jürgen Prof. Dr., 73457 Essingen (DE); Lingelbach, Bernd Prof. Dr., 73453 Abtsgmünd (DE)

(57) **Zusammenfassung**

Die Erfindung erlaubt die Schaffung einer kostengünstig herstellbaren Vorrichtung zur automatisierten Messung der Form spiegelnder sphärischer oder auch asphärischer Flächen. Der zu vermessenden Fläche wird ein beleuchtetes Muster gegenübergestellt oder auf sie projiziert, wobei das über die spiegelnde Fläche entstehende Bild des Musters von einer elektronischen Kamera festgehalten wird. Wesentlich ist, dass dieses Muster so gezeichnet ist, dass sein auf dem Bildsensor der Kamera entstehendes Bild ein System von annähernd geraden und äquidistanten Streifen darstellt. Derartige Bilder lassen sich z.B. mittels der diskreten Fouriertransformation mit besonders hoher Genauigkeit auswerten. Will man die spiegelnde Fläche nicht nur längs einer Schnittkurve, sondern insgesamt vermessen, wählt man vorzugsweise ein derartiges Muster, das auf dem Bildsensor zwei solche Systeme äquidistanter und gerader Streifen ergibt, wobei vorzugsweise die auf dem Bildsensor entstehenden Streifensysteme zueinander orthogonal sind.
Die untere Figur zeigt ein Ausführungsbeispiel. Mögliche Ausführungen für stärker gekrümmte spiegelnde Flächen sind im Text und in Zeichnungen dargestellt.
In manchen Anwendungsfällen ist es sinnvoll, zugleich den Abstand der spiegelnden Fläche zur Messvorrichtung zu kontrollieren. Daher sind auch Ausführungsbeispiele einer Kombination mit einem optischen Abstandsmesssystem dargestellt.

## Beschreibung

Die Erfindung betrifft eine Vorrichtung entsprechend dem Oberbegriff des Anspruchs 1.

Bei der Herstellung optischer Linsen und Spiegeln und für die ärztlichen Diagnostik der Hornhaut des Auges oder auch für die Anpassung von Kontaktlinsen ist die Vermessung der Flächenform zusammen mit einer rechnergestützten Auswertung wünschenswert.

Spiegelnde Flächen können bekanntlich mit Interferometern sehr genau vermessen werden. Im Fall von stark gekrümmten und asphärischen Flächen wie bei einer Kontaktlinse oder Hornhaut ist dies prinzipiell möglich (Deutsche Optiker Zeitung, 8 (1994) S. 108), jedoch ist eine spezielle Korrekturoptik erforderlich, welche die Kosten des ohnehin teuren Systems weiter erhöht.

Durch das Patent US 4 792 232 ist eine Vorrichtung zur Prüfung spiegelnder Flächen bekannt geworden, bei dem man der Fläche ein Strichgitter gegenüber steht und die Kanten des an der Messfläche gespiegelten Bilds auswertet. Für stark gekrümmte Flächen ist diese Anordnung jedoch ungeeignet, da im gespiegelten Bild die Striche auf einer Seite extrem eng beieinander und auf der gegenüberliegenden Seite sehr weit voneinander entfernt liegen würden.

Für die Vermessung der Hornhaut sind seit langem Keratometer bekannt, die auf dem Prinzip der Placido-Scheibe beruhen. Hierbei stellt man dem Auge eine Scheibe mit konzentrischen hellen Ringen oder auf Kreisringen angeordneten Leuchtpunkten gegenüber und untersucht das vom Tränenfilm auf der Hornhaut gespiegelte Bild der Scheibe. In der früheren Vergangenheit wurden die Bilder photographisch festgehalten und manuell ausgewertet (Augenoptik, Band 86, (1969) S. 69). Viele derzeit auf dem Markt angebotene Keratometer beruhen auf der Auswertung des Reflexes einer Placido-Scheibe (Die Kontaktlinse 1 - 2 (1996) S. 5). Die Auswertung durch einen angeschlossenen elektronischen Rechner erlaubt Aussagen über Krümmungsradien der Hornhaut oder versucht die Flächenform durch Anpassen vorgegebener mathematischer Funktionen zu beschreiben. Details der Flächenform können nicht dargestellt werden.

Weiterhin sind Verfahren zur Vermessung der Hornhaut bekannt geworden, die auf der Rasterstereographie beruhen (Applied Optics, Band 27 (1988) S. 1135) Ein Muster paralleler und äquidistanter Streifen wird mit blauen, Licht auf die Hornhaut projiziert und dem Tranenfilm wird durch Eintröpfeln ein Fluoreszenzstoff beigegeben. Im Fluoreszenzlicht erscheinen die Streifen dann gekrümmt und mit unterschiedlichen Abständen. Die Oberflächenform wird aus der Verformung des Streifenmusters errechnet. Nachteilig ist hierbei die Notwendigkeit der Zugabe des Fluoreszenzmittels, das oftmals die Patienten irritiert und für den Arzt ausserdem einen zusätzlichen Zeitaufwand bedeutet.

Die Aufgabe der Erfindung ist es, eine kostengünstige Vorrichtung zu schaffen, welche die Form stark gekrümmter spiegelnder Flächen mit hoher Genauigleit und mit guter Wiedergabe von Details messen kann.

Diese Aufgabe wird durch eine Vorrichtung mit den Merkmalen des Anspruchs 1 gelöst.

Aus der Interferometrie ist bekannt, dass Muster von geraden und äquidistanten Streifen mit sinusförmigem Helligkeitsverlauf mit hoher Genauigkeit ausgewertet werden können (Applied Optics, Band 26 (1987) S. 1668). Gemäß der Erfindung wird der spiegelnden Fläche ein beleuchtetes Muster gegenübergestellt, so wie es auch in den auf der Placido-Scheibe beruhenden Keratometern verwirklicht ist. Das Muster kann auch durch ein optisches System auf die Messfläche projiziert werden. Erfindungsgemäß verwendet man jedoch ein Muster, welches derart gezeichnet ist, dass sein über die spiegelnde Fläche und das Kameraobjektiv auf dem Bildsensor erzeugtes Bild eine Folge möglichst gerader und äquidistanter Streifen mit sinusförmigem Helligkeitsverlauf darstellt. Dieses Bild wird gespeichert und von einem elektronischen Rechner ausgewertet. Hierbei wird zunächst die Streifenordnungszahl für alle Punkte auf dem zu den Streifen orthogonal liegenden Meridian bestimmt und daraus der Neigungswinkel jedes Flächenelements auf dem Meridian berechnet. Durch numerische Integration längs des Meridians lässt sich dann die Schnittkurve ermitteln.

Aufgrund der Geradheit und Äquidistanz der Streifen im Bild des Musters ist es im Raumfrequenzbereich sehr gut konzentriert und kann daher mittels der diskreten Fouriertransformation und digitaler Filterung leicht isoliert werden. Der Helligkeitsverlauf des Musterbilds muss daher nicht perfekt sinusförmig sein, da eventuell auftretende Oberwellen des abgebildeten Musters durch die Filterung im Raumfrequenzbereich unterdrückt werden können.

Will man die gesamte Fläche vermessen so kann man das oben beschriebene Verfahren verwenden und durch Drehen des Prüflings oder des Musters die Schnittkurven für mehrere Meridiane zu bestimmen. Um jedoch mit nur einer Messung die gesamte Flachenform zu ermitteln benutzt man zweckmäßigerweise ein derart gezeichnetes Muster, das auf dem Bildsensor eine Überlagerung zweier zueinander orthogonaler Systeme von äquidistanten geraden Streifen ergibt. Mittels der diskreten Fouriertransformation des Bildes und digitaler Filterung im Raumfrequenzbereich lassen sich die beiden zueinander orthogonalen Streifensysteme trennen und einzeln auswerten. Damit kann für jeden Punkt der Messfläche ermittelt werden, von welchem Punkt des Musters der Strahl ausging. Hieraus können aufgrund des Reflexionsgesetzes für jeden Punkt der Messfläche die Neigung der Tangentialebene errechnet werden, woraus man dann durch numerische Integration die Form der gesamten Oberfläche ermitteln kann.

Für eine grobe visuelle Kontrolle kann das Muster über die spiegelnde Fläche und ein Objektiv auf ein Transmissionsgitter anstelle des Bildsensors abgebildet werden, so dass Moiré-Streifen auftreten, und eine grobe Abweichung der Flächenform von einer Sollform visuell überprüfbar ist.

Zur Erstellung des Musters geht man zweckmäßigerweise folgendermaßen vor: In der Regel ist die Sollform der zu vermessenden Fläche bekannt. Im Falle der Hornhaut könnte dies beispielsweise ein Rotationsellipsoid mit einem Krümmungsradius im Apex von 7.5 mm und einer numerischen Exzentrizität von 0,4 sein. Man berechnet anhand der Sollform für jeden Punkt des Musters den zugehörigen Bildpunkt auf dem Bildsensor und die Bildhelligkeit, die an dieser Stelle vorliegen soll. Der entsprechende Grauwert wird dem betreffenden Punkt des Musters zugeordnet. Dieses Verfahren lässt sich mit einem elektronischen Rechner automatisiert durchführen. Mit diesen Daten kann ein Elektronenstrahlschreibgerät eine Belichtungsmaske erzeugen und die gewünschten Muster können mittels Reprografie hergestellt werden. Bei geringeren Anforderungen an die Messgenauigkeit genügt auch ein von einem rechnergesteuerten Drucker gezeichnetes Muster. Eine weitere interessante Variante ist, das Muster durch einen rechnergesteuerten Bildschirm zu erzeugen. Auf diese Weise kann man im Falle von Messflächen sehr unterschiedlicher Form leicht das jeweils optimale Muster auswählen und zur Messung verwenden.

Der Vorteil der erfindungsgemäßen Lösung gegenüber der interferometrischen Methode besteht klarerweise in dem erheblich geringeren Aufwand. Im Vergleich zu den auf der Placido-Scheibe beruhenden Keratometern ist von der erfindungsgemäßen Lösung aus dem folgenden Grund eine höhere Genauigkeit und bessere Deatilwiedergabezu erwarten: Wendet man Muster von Kreisen oder radialen Linien an, so können für die Bildauswertung nur Punkte, die eine Linienmitte oder den Rand eines Kreisrings darstellen. verwendet werden. Bei dem hier beschriebenen Verfahren wird hingegen die in der Helligkeit jedes Bildpunkts enthaltene Information genutzt. Die Form der Messfläche wird nicht durch Anpassen der Parameter mathematischer Funktionen beschrieben. sondern wird punktweise voranschreitend errechnet.

### Ausführungsbeispiele

Im folgenden werden einige Ausführungsbeispiele der Erfindung in Text und Zeichnungen dargestellt.

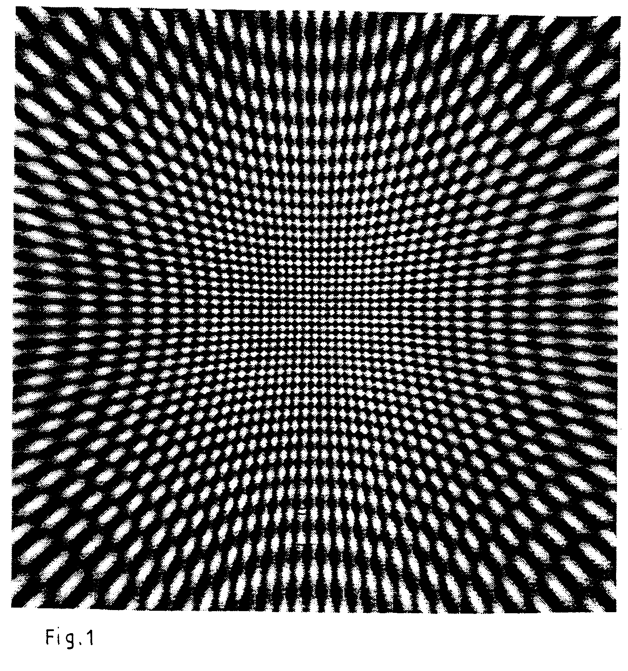

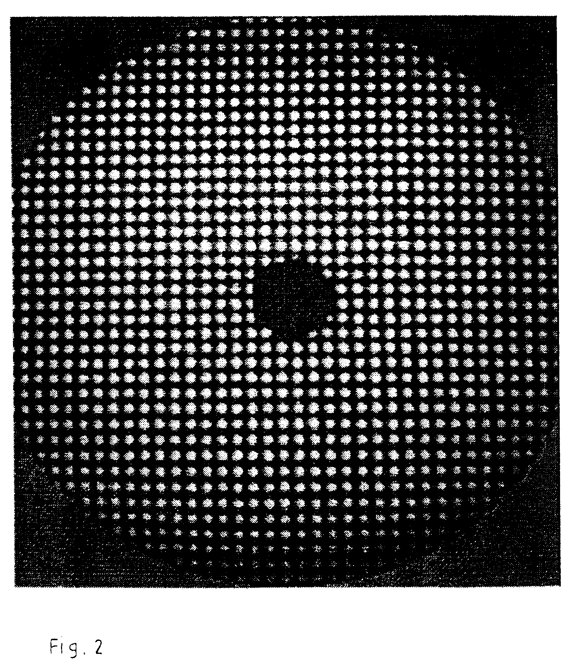

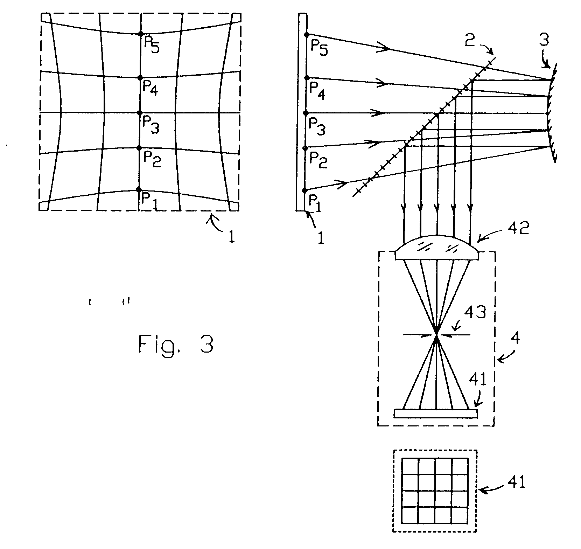

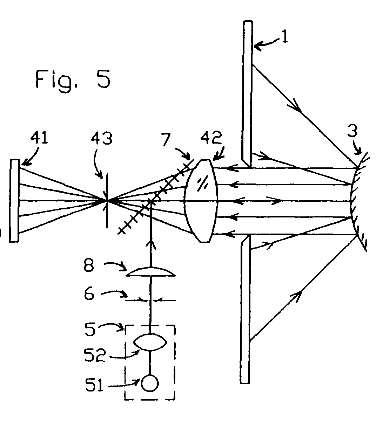

Zur Illustration zeigt die Figur 1 ein Muster, das für die Vermessung einer Kontaktlinsenfläche mittels einer Vorrichtung gemäß Figur 5 gezeichnet wurde. Die Figur 2 gibt das an der Kontaktlinsenfläche gespiegelte Bild des Musters wieder. Die Figuren 3 bis 6 zeigen schematisch Beispiele geeigneter Anordnungen. Die gezeigten Strahlen stellen jeweils den Hauptstrahl des von dem jeiligen Punkt des Musters ausgehenden Strahlenbündels dar. Auf die Darstellung von Halterungen der Baugruppen oder von Vorrichtungen zur Durchleuchtung oder Beleuchtung der Muster wurde verzichtet.

Das in Figur 3 dargestellte Ausführungsbeispiel eignet sich vorzugsweise zur Messung von wenig stark gekrümmter technischen Flächen oder für die Untersuchung eines kleinen Ausschnitts der Hornhaut. Das Muster (1) ist auf einem ebenen Träger aufgebracht. Die von den Punkten P₁ bis P₅ des Musters ausgehenden Strahlen durchtreten den teildurchlässigen Spiegel (2) um dann auf die spiegelnde Fläche (3), fortan auch Messfläche genannt, zu treffen. Nach der Reflexion gelangen die Strahlen wieder zum teildurchlässigen Spiegel (2) der sie zur Kamera (4) hin reflektiert. Auf dem Bildsensor (41) der Kamera entsteht dann das Bild des Musters (1). Die Öffnungsblende (43c) ist in der Bildbrennebene des Objektivs (4) angeordnet, sofass sich ein telezentrischer Strahlengang ergibt. Ein telezentrischer Strahlengang vereinfacht die rechnerische Auswertung, ist jedoch nicht zwingend. Klarerweise könnten in einer Anordnung gemäß der Figur 3 auch die Positionen des beleuchteten Musters (1) und der Kamera (4) vertauscht sein, so dass die Strahlen vom Muster über eine Reflexion am teildurchlässigen Spiegel (2) zur Messfläche (3) gelangen und von dort durch den teildurchlässigen Spiegel (2) zur Kamera (4) hindurchtreten.

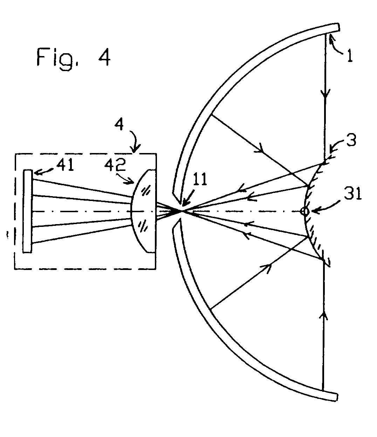

Die Figur 4 zeigt eine für stark gekrümmte Flächen geeignete Anordnung. Da aufgrund der großen Öffnung der Strahlenbündel ein teildurchlässiger Spiegel nicht mehr anwendbar ist, werden die Kamera (4) und das Muster (1) koaxial vor der Messfläche (3) aufgebaut. Das Muster (1) ist auf einem kugelförmigem Träger aufgebracht, der eine zentrale Öffnung (11) besitzt, durch welche die von der Messfläche (3) gespiegelten Strahlen zur Kamera (4) hindurchtreten können. Zweckmäßigerweise wild das Objektiv (42) der Kamera so gebaut, dass die Öffnung (11) zugleich als Öffnungsblende der gesamten Abbildung wirkt. Dann kann man bei genügender Beleuchtung des Musters die Öffnung (11) klein dimensionieren so dass nur innerhalb einer kleinen Umgebung des Scheitelpunkts (31) die Form der Messfläche nicht direkt gemessen werden kann. Sie kann jedoch durch Extrapolation aus den benachbarten Bereichen rechnerisch weitgehend rekonstruiert werden.

Für eine genaue Bestimmung der Flächenform muss der Abstand der Messfläche (3) der vom Objektiv (42) möglichst genau bekannt sein. Bei schwer fixierbaren oder mechanisch nicht antastbaren Messflächen wie beispielsweise der menschlichen Hornhaut oder weichen Kontaktlinsen kann das Einhalten des korrekten Abstands über die Schärfe des Bilds des Musters überprüft werden. Hierfür kann man die Öffnungsblende der Kamera als Springblende, wie es von einäugigen Spiegelreflex-Kameras bekannt ist, ausführen. Vor der Aufnahme wird die Blende weit geöffnet, so dass aufgrund der geringen Schärfentiefe der Abstand sehr genau eingestellt werden kann. Beim Auslösen der Aufnahme wird die Blende auf einen geringen Durchmesser zugezogen, um das Bild des Musters mit großer Schärfentiefe festzuhalten.

Figur 5 zeigt eine Ausführung zur Vermessung der gesamten Hornhaut. Das Muster (1) ist auf einem ebenen Träger mit einer zentralen Öffnung aufgebracht. Die Kamera ist mit einem telezentrischen Messobjektiv ausgestattet. Wie bei der in Figur 4 gezeigten Ausführung kann ein kleiner zentraler Bereich der Hornhaut nicht vermessen werden. Dieser Bereich wird aber für die Kontrolle des richtigen Abstands der Hornhaut vom Muster genutzt. Hierfür wird mittels einer Beleuchtungseinrichtung (5), die aus einer Lichtquelle (51) und einem Kondensor (52) besteht, eine Blende (6), die eine sehr kleine kreisförmige Öffnung besitzt, durchleuchtet. Die Strahlen gelangen von der Öffnung der Blende (6) über den teildurchlässigen Spiegel (7) und das Objektiv (42) auf den zentralen Bereich der Hornhaut. Durch Einfügen einer Zylinderlinse (8) wird, wie bei dem in CD-Spielern verwendeten Autofokussystem, eine astigmatische Abbildung der Blende (6) auf den Bildsensor (41) erreicht. Die Lagen der Zylinderlinse (8) und der Blende (6) werden so gewählt, dass bei korrektem Abstand der Hornhaut das Bild der Blende (6) auf dem Bildsensor (41) als kreisförmige Streufigur erscheint. Bei zu großem oder zu kleinem Abstand der Messfläche erscheint eine liegende oder eine stehende Ellipse als Streufigur. Als weiterer Nutzen kann die beleuchtete Blende zugleich als Fixationslicht für den Probanden dienen.

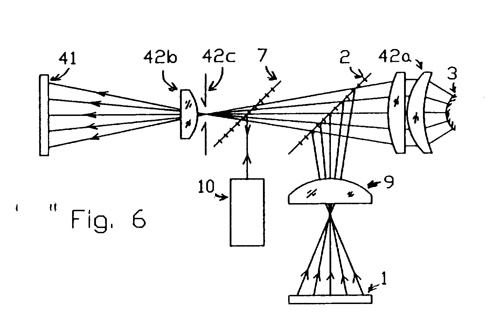

Die Figur 6 zeigt das Schema einer aufwendigeren Ausführung, die es gestattet, die gesamte Hornhaut einschließlich des zentralen Bereichs zu vermessen. Das Muster (1) wird über ein zusätzliches Objektiv (9), den teildurchlässigen Spiegel (2) und der vorderen Gruppe (42a) des Kameraobjektivs, das eine hohe numerische Apertur besitzt, auf die Hornhaut (31) projiziert. Das von der Hornhaut (31) gespiegelte Bild des Musters (1) wird von dem aus den Baugruppen (42a) und (42b) bestehenden Kameraobjektiv auf den Bildsensor (41) abgebildet. Es ist hier vorteilhaft, das Kameraobjektiv in die Baugruppe (42a), die das an der Hornhaut gespiegelte Muster nach unendlich abbildet, und die Baugruppe (42b), die das Muster auf den Bildsensor abbildet aufzutrennen, da dann der teildurchlässige Spiegel (2) im parallelen Strahlengang keine Abbildungsfehler verursacht. Zur Kontrolle des korrekten Abstands der Hornhaut (3) von der Bugruppe (42a) des Kameraobjektivs kann eine Vorrichtung zur optischen Entfernungsmessung oder ein Abstandssensor (10) eingesetzt werden, dessen Strahlengang über den teildurchlässigen Spiegel (7) eingekoppelt wird. Die Strahlung des Abstandssensors kann zugleich als Fixationslicht dienen. Um eine Störung des von der Kamera (4) aufgenommenen Bilds durch die Strahlung des Abstandssensors (10) zu verhindern, kann man das Muster (1) mit Strahlung anderer Wellenlängen als die für den Abstandssensor (10) verwendete Lichtwellenlängen beleuchten und den teildurchlässigen Spiegel (7) als Farbteiler ausführen. Als weitere Möglichkeit kann man die Strahlungsquelle des Abstandssensors für die Dauer der Aufnahme abschalten. Zur vereinfachten Handhabung des Keratometers kann die Aufnahme des Musterbilds durch ein bei korrektem Abstand der Hornhaut (3) erfolgendes Signal des Autofokussensors (10) automatisch auslösen lassen. Falls durch krankhafte Veränderungen bedingte extrem ungewöhnlich geformte Hornhäute untersucht werden sollen, kann es vorteilhalt sein, ein jeweils optimal abgestimmtes Muster (1) zu verwenden. Um schnell zu einem anderen gewünschten Muster (1) zu kommen, kann das Muster (1) durch einen elektronischen Bildschirm, beispielsweise ein durchleuchteter Flüssigkristall-Bildschirm, erzeugt werden. Dieser elektronische Bildschirm kann von einem elektronischen Rechner gesteuert werden.

Für Anwendungen zur Vermessung der Hornhaut können auch die in den Figuren 3 und 4 skizzierten Vorrichtungen mit einem Fixationslicht oder einem Autofokussensor versehen werden. Die Strahlung des Fixationslichts oder des Autofokussensors kann über einen zwischen dem Muster (1) und dem Kameraobjektiv (42) eingefügten teildurchlässigen Spiegel eingekoppelt werden.

## Patentansprüche

1. Verfahren zum optischen Messen der Form spiegelnder Flächen, mit den folgenden Merkmalen:
1.1 der spiegelnden Fläche wird ein beleuchtetes Muster gegenübergestellt;
1.2 das Muster ist derart gestaltet, daß sein über die spiegelnde Fläche erzeugtes Bild ein System gerader und äquidistanter Streifen mit sinusförmigem Helligkeitsverlauf darstellt.

2. Verfahren nach Anspruch 1, gekennzeichnet durch eine solche Gestaltung des Musters, daß das erzeugte Bild zwei oder mehrere Streifensysteme aufweist.

3. Verfahren nach Anspruch 1 oder 2, dadurch gekennzeichnet, daß ein optisches System zwischen Muster und spiegelnder Fläche geschaltet wird.

4. Verfahren nach einem der Ansprüche 1 bis 3, dadurch gekennzeichnet, daß das Muster über die spiegelnde Fläche auf den Bildsensor einer elektronischen Kamera abgebildet wird.

5. Verfahren nach einem der Ansprüche 1 bis 4, dadurch gekennzeichnet, daß das Muster über die spiegelnde Fläche auf ein Gitter oder auf ein Kreuzgitter zur Erzeugung eines Moiré-Musters abgebildet wird.

6. Verfahren nach einem der Ansprüche 1 bis 5, dadurch gekennzeichnet, daß der Strahlengang zwischen Muster und spiegelnder Fläche, und der Strahlengang zwischen spiegelnder Fläche und der Kamera über einen teildurchlässigen Spiegel vereinigt werden.

7. Verfahren nach einem der Ansprüche 1 bis 5, dadurch gekennzeichnet, daß das beleuchtete Muster und die Kamera koaxial gegenüber der spiegelnden Fläche angeordnet werden, und daß das Muster eine Öffnung für den Strahlengang zwischen spiegelnder Fläche und Kamera besitzt.

8. Verfahren nach einem der Ansprüche 1 bis 7, dadurch gekennzeichnet, daß zusätzlich eine Vorrichtung zur Positionserkennung oder zur Abstandsmessung der spiegelnden Fläche oder ein Fixationslicht eingebaut werden.

9. Verfahren nach Anspruch 8, dadurch gekennzeichnet, daß das Positionserkennungs- oder das Abstandsmessungssystem automatisch die elektronische Kamera auslöst.

10. Verfahren nach Anspruch 8 oder 9, dadurch gekennzeichnet, daß ein Farbteiler für die Einkoppelung der Strahlung des Positionserkennungs- oder Abstandsmessungssystemes oder eines Fixationslichtes verwendet wird.

11. Verfahren nach Anspruch 8 oder 9, dadurch gekennzeichnet, daß während der Kamerabelichtung die Lichtquelle des Positionserkennungs- oder Abstandsmessungssystemes oder des Fixationslichtes abgeschaltet wird.

12. Verfahren nach einem der Ansprüche 1 bis 11, dadurch gekennzeichnet, daß eine schnell verstellbare Öffnungsblende verwendet wird.

13. Verfahren nach einem der Ansprüche 1 bis 12, dadurch gekennzeichnet, daß das Muster durch einen elektronisch gesteuerten Bildschirm erzeugt wird.
